# EUROPEAN PATENT APPLICATION

(11) **EP 2 886 195 A1**
(43) Date of publication of application: **24.06.2015**
(21) Application number: 13306829.6
(22) Date of filing: 23.12.2013
(51) Int. Cl.: B01L 3/00, C12Q 1/02, G01N 35/08

(54) **Method for determining the effect of chemical substances on microorganisms**

(71) Applicant: Rhodia Operations, 93306 Aubervilliers Cedex (FR); Université de Bordeaux 1, 33076 Bordeaux (FR); Centre National de la Recherche Scientifique, 75794 Paris Cedex 16 (FR)
(72) Inventor: COLIN, Annie, 33000 Bordeaux (FR); GUILLOT, Pierre, 33600 Pessac (FR); MARCHAL, Philippe, 69230 Saint-Genis Laval (FR); LALANNE-AULET, David, 33000 Bordeaux (FR)
(74) Representative: Vande Gucht, Anne

(57) **Abstract**

A method for determining the effect of chemical substances on microorganisms in miniaturized devices

## Description

The present invention relates to a method for determining the effect of chemical substances on microorganisms in miniaturized devices

Controlled operations of liquid systems at the micro or milli scale are more and more replacing standard chemical and biological assays in laboratories. The various types of tests which have usually been carried out in millilitre and microlitre volumes are now available in nanolitre or picolitre droplets.

Droplet-based fluidics provides a technological upgrade of the compartmentalization concept which has been used e.g. for genome sequencing applications. Typical for this approach is the localization of reactants and products which leads to high product concentrations owing to small volumes. Thus, encapsulation of microbial populations into droplets and the monitoring of their behaviour is of great interest for fundamental microbiology and clinical tests.

Baraban et al., Lab Chip, 2011, 11, 4057 disclose a millifluidic droplet analyzer for microbiology that is designed to perform a variety of precise clinical and microbiological assays of microbial populations in replicate droplets. The device represents a scale-up from the microfluidic approach, operating with larger droplets, but maintains the fundamental principles of fluid control and high throughput. In contrast to microfluidic approaches working with picolitre reservoirs, the droplets in millifluidic systems of the reference have volumes of around 100 nL, which allows the switching from micro-channels to commercially available tubes, e.g. based on fluorinated ethylene propylene tubes with an inner diameter of approximately 0.5 mm. The key feature of the device described is the assembly of various modules with a specific function like in microfluidic systems, but with fluidic components used in the field of chromatography. The droplet analyzer of the reference operates more than 1000 separate water-in-oil droplets that act as mini-incubators for the encapsulated living cells. The growth of the microorganism in the droplets is studied via the detection of a fluorescent signal. Droplets are produced in a cross-junction geometry and form a one-dimensional train where each droplet represents an element of a numeric sequence. The order of the droplets is preserved during the experiment which leads to a self-labelling of each aqueous reservoir that contains the microorganisms.

The drop maker module forms a large number of isolated aqueous droplets containing microorganisms in predefined microenvironments. Solutions of microorganisms, nutrients and antibiotics are prepared and stored in three separate syringes. These solutions are then injected by independent high-precision syringe pumps and are then mixed at a cross junction A. The train of uniform droplets containing bacteria and dispersed in a fluorinated oil is formed at a cross junction B. Spacer droplets of mineral oil can be injected at cross junction B to spatially separate the neighbouring bacterial populations. This step is considered important because of the high density of the droplets which causes the appearance of so-called traffic jams in the tubes and may lead to the possible overlapping of the fluorescent signals from neighbouring bacterial populations during detection.

The detection module consists of a fluorescence detector and a so-called back-and-fourth circuit. Because the detection point is fixed, the motion of the droplets train towards the detector has to be initiated by injecting the continuous phase at a given flow rate. The droplets are therefore sampled one by one at a fixed frequency. When the scan of the forward train of droplets in the forward direction is completed, a valve is automatically switched, initiating reverse motion of the droplet train in the circuit and providing a subsequent back run of drop sampling.

Cira et al., Lab Chip 2012, 12, 1052, discloses a self-loading microfluidic device for determining the minimum inhibitory concentration of antibiotics. The reaction chambers of the microfluidic device before sealing are filled with different concentrations of antibiotic and dried. After sealing the device a droplet of the solution containing the microorganism is put on the inlet port of the device the chambers of which are loaded by a self-loading mechanism using degas driven flow. The solution containing the microorganism dissolves the antibiotic in the various chambers and the reliability of the measurement clearly depends on the dissolution of the entire amount of antibiotics in each chamber without intermixing between the different chambers. Thus, the final incubator is formed within the respective chambers.

Takagi et al., Analyst 2013, 138, 1000, describes a microfluidic microbioculture device for rapid determination of the Minimum Inhibitory Concentration (MIC) of antibiotics where a small volume of sample solution is introduced into multiple chambers simultaneously and the growth of bacteria is quantified using a non-invasive three-dimensional visualization technique. Similar as in Cira et al., the antibiotics are immobilized as a freeze-dried matrix formed in the chambers to realize homogeneous mixing of the components after the introduction of the cell suspension solution.

While the above-described methods and devices of the prior art present a progress to the classical analytical methods, they are still not fully satisfactory for high-precision measurements in high-throughput systems.

There thus exists an ongoing need for methods for determining the effect of chemical substances on microorganisms in miniaturized devices with high precision and high speed, which could also follow effects of chemical substances on growth curve kinetic.

It was thus an object of the present invention to provide a method for determining the effect of chemical substances on microorganisms in miniaturized devices which overcomes the deficiencies of the prior art systems.

This object is achieved by the method in accordance with claim 1. Preferred embodiments of the present invention are set forth in the dependent claims and in the detailed specification and the working examples hereinafter.

In accordance with the method of the invention, the effect of a chemical substance on microorganisms is monitored in a miniaturized device. The method allows high-throughput screenings as well as long-time monitoring of the effect and thus is flexible and can be adapted to a great variety of different application needs.

In one embodiment, the method of the invention is using the incorporation of microorganisms in mini-reactors or mini-incubators in the form of droplets which contain the analyte and the chemical, the latter in different concentrations, and, a nutrient system (broth media) that can support the growth of microorganisms. The evolution of the population of the microorganisms under the influence of the chemical substance is then monitored The method in accordance with the present invention can be used for MIC evaluation.

The method of the invention allows to monitor the effects of chemical substances on microbial growth microorganisms in droplets in a nondestructive or non-intrusive manner, i.e. without interfering physically with the droplet reaction system.

Another application of the system is the miniaturization of challenge test in droplets which contain the formulation to be tested and the microorganisms. In that case, a nutrient system (broth media) is not necessary.

In the first step of the method in accordance with the invention, aqueous droplets comprising the microorganisms, a nutrient system, if necessary, and the chemical substance or the formulation under investigation are formed outside the miniaturized device, i.e. the mini-reactors or mini-incubators in the form of the aqueous droplets are formed before transferral into the miniaturized device. This provides the advantage that the composition of the mini-reactors or mini-incubators (each droplet represents one mini-incubator or mini-reactor) can be precisely controlled. Compared to the devices of the prior art and the methods using such devices where one component of the reaction system is pre-stored in the device this has the advantage that it can be assured that all components of the composition are present in a reproducible amount and without the risk of having undergone changes during the pre-storage time in the known devices. This is advantageous for reliable and reproducible measurements. In the case of formulations, the possibility to create numerous formulations with a broad variety of compositions and ingredients with the device is of great interest.

The method of the present invention is principally suitable for following the effect of chemical substances or formulations on any microorganism. Only be way of example, bacteria, yeasts and algae, in particular unicellular algae may be mentioned here.

Preferably, the method of the present invention is carried out under aseptic conditions (sterilization of media, pre-culturing of the microorganisms under sterile conditions etc.). The skilled person is aware how to achieve these conditions to exclude the influence of external impurities or components and will provide and select the respective conditions.

The formation of droplets in step a) of the present invention can be achieved by methods principally known to a skilled person. For example, the different components (chemical substance or formulation, microorganisms and, if necessary, nutrient system (broth)) can be prepared separately and stored in separate reservoirs from which they are transferred and mixed into the droplets functioning as mini-reactor or mini-incubator. Suitable reservoirs could be syringes or the like which allow the easy transfer and mixing at a cross junction. The mixing of the components and the formation of the droplets can preferably be achieved by using high-precision syringe pumps for injecting and mixing the different solutions at a suitable cross junction. The high precision syringe pumps allow the exact dosing of each component in the desired amount with very high precision, which is essential for the method of the present invention. In the case of formulations and miniaturisation of challenge test, the possibility to create numerous formulations with the syringe pumps device is of great interest. Due to the small volumes of the mini-incubators, small variations in the amount of the components can have a significant change of the ratio of the components as a consequence which is undesirable for quantitative measurements.

Other means for the exact dosing of small volumes known to the skilled person are also suitable for formation of the droplets in step a) of the method of the invention. The person skilled in the art knows such means and will select suitable means based on his professional knowledge and the specific application case.

In the second step of the method in accordance with the present invention, a one dimensional train of the droplets obtained in step a) is formed in a carrier based on a fluorinated oil. The fluorinated oil carrier allows the separation of the droplets or mini-reactors (mini-incubators) from each other in the droplet stream, i.e. it allows to avoid intermixing between different droplets which would be disadvantageous. Each droplet being a separate mini-reactor with a defined reaction system (characterized by a certain ratio of the amount of the components), intermixing between droplets would lead to irreproducible results.

The droplets formed in step a) of the method of the present invention can be mixed with the hydrophobic oil carrier at a cross junction B thereby forming the desired train of alternating drops of mini-reactors and hydrophobic oil. As for step a, suitable means for achieving such mixing without intermixing the droplets of the two components are known to the skilled person and he will select appropriate means based on his professional experience depending on the specific type of reaction system.

In accordance with a preferred embodiment of the method of the present invention, the one dimensional train of droplets is formed by mixing the droplets formed with the hydrophobic oil in a suitable mixing chamber allowing the formation of discrete aqueous droplets isolated from each other in the hydrophobic oil carrier.

The volume of the single droplets is not subject to particular limitations and will be chosen such that, depending on the device, where the droplets are immobilized in subsequent step c) a suitable droplet size will be chosen allowing secure transfer on one hand and good immobilization on the other hand. Thus, droplet size or diameter and channel size or tube diameter should fit with each other to allow secure immobilization. It has been proven advantageous in a number of cases if the droplet size is slightly exceeding the diameter of channels in the miniaturized device or the cross section of the channels of microfluidic chips to ensure that no movement of the droplets along the tube during the monitoring takes place.

Preferably, the sample volume (volume of the droplets is in the range of from 0.1 nL to 50 µL, preferably in the range of from 0.5 nL to 25 µL and particularly preferably in the range of from 1 nl to 10 µL.

A preferred group of hydrophobic oils suitable for use in the method of the present invention are fluorinated oils like. Suitable oils are available commercially from a number of suppliers, including Solvay S.A., Minnesota Mining and Manufacturing company or Du Pont de Nemours, to mention only a few suppliers. Exemplary tradenames of the respective products are Fomblin^{®} PFPE or Galden^{®} PFPE (PFPE stands for polyperfluoropolyether) from Solvay SA, which represent preferred fluorinated oil products. Another example is HFE 7500 from Minnesota Mining and Manufacturing Company).

In accordance with a further preferred embodiment of the method in accordance with the present invention, the formation of the one dimensional train is achieved in the absence of mineral oil which is used as a spacer in Baraban et al. (2011) disclosed above. The mineral oil bears the risk that an interaction might occur with the droplets which could influence the measurement.

In step c) of the method in accordance with the present invention, the one dimensional train of droplets isolated from each other is transferred into a miniaturized device, thereby immobilizing the droplets in the device.

Immobilizing the droplets is intended to mean that the one dimensional train of droplets is not subject to movement within the device after the transfer. Thus, different to the device disclosed by Baraban et al. (2011) discussed above, the droplets are not moving in the device in changing directions in the course of the analysis. Each and every droplet maintains a constant and fixed relative position within the device during the entire period of monitoring. There is thus no risk of intermixing between droplets (or mini-reactors) as present in Baraban et al (2011) where a flow of the droplets in alternating directions within the device occurs.

The miniaturized devices suitable for use in the method of the present invention are not subject to particular restrictions and thus and such devices capable of securing a constant position of the droplets are in principle suitable.

A first preferred example of such devices are miniaturized tubes, preferably based on inert materials such as fluorinated polymers which have a diameter usually in the range of from 0.05 to 5 mm, preferably of from 0.2 to 2.5 mm. The train of droplets can be introduced into such tubes which are sealed at one end and after the tube is filled with the train of droplets, the tube is also sealed at the open end thereby rendering a sealed tube comprising a multiplicity of mini-reactors or mini-incubators which , due to the sealing at both ends have a constant position within the tube which allows a reproducible allocation of each single mini-reactor or mini-incubator within the tube which is particularly advantageous for long term monitoring as each mini-reactor can be assigned a specific identifier based on the position in the tube which allows automated monitoring of the mini-reactors or mini-incubators with the possibility to automatically assign a signal to a specific mini-incubator. Systems where the mini-reactors are moving for the monitoring always bear the risk that relative positions change in the course of the monitoring which makes automatic monitoring difficult.

Another example of preferred devices for storing the droplets (mini-reactors) and monitoring the effect of the chemical substances on the microorganisms are so called micro-fluidic devices in the form of so called microfluidic chips. In these micro-fluidic chips the flow of liquid takes place in micro-channels with small cross-sections usually in the range of from 1x1 µm² to 500x500 µm². The one dimensional train is transferred into such devices in a similar manner as described above for the tubes and the immobilization of the droplets (mini-reactors or mini-incubators) is achieved through three dimensional inserts in the micro-channels or by forming micro-reaction chambers which can hold one droplet.

The two preferred devices described above are only exemplary for suitable devices. In principle any device known to the skilled person which allows the reliable immobilization of the mini-reactors or incubators in the system can be used. The skilled person is aware of respective devices and will select the best suitable device based on the actual need.

Due to the immobilization of the mini-reactors or incubators, no flow in the system during the period of monitoring is needed. Thus, a series of devices could be used, each one supporting several test series which could be put in front of the detection device only from time to time, e.g. once a day or even once a week or longer, which allows long term studies not easily possible with systems where a flow of the mini-reactors is involved. Closed tubings can be easily stored for such long term experiments. A continuous flow system as has been described in the prior art is not well suited for such measurements.

The final step of the method comprises repeatedly measuring a signal indicative of the microorganism growth for each of the droplets in defined time intervals (which can span a very broad range of time as outlined above.

The signal is preferably measured in a non-invasive manner thereby avoiding an interference of the detection system with the mini-reactors or mini-incubators by physical contact.

In principle any signal indicative of the growth behaviour or the microorganism without needing physical contact with the reactor is suitable. Fluorescence methods, light diffusion methods, optical counting (e.g. by bright field imaging of the droplets) or combinations of fluorescence detection with absorbance measurements may be mentioned here by way of example.

A combination of fluorescence detection and absorbance measurement has proven to be particularly useful in certain cases. The advantage of coupling or combining both methods allows a quantitative monitoring during the whole growth which allows to obtain quantitative kinetic information and fully comparable results towards usual tests.

The skilled person is aware of and familiar with the respective methods and will select the best suitable method based on the individual needs.

Certain microorganisms show a natural fluorescence (i.e. algae) and basically all microorganisms can be genetically modified to show fluorescence without influencing their growth and reproduction behaviour.

Dynamic light diffusion, i.e. temporary fluctuations of the diffusion intensity may also be used to monitor the reaction in the mini-reactors or mini-incubators.

Light diffusion measurements are principally carried out as follows: The mini-reactor is irradiated with a laser at an angle φ which defines the angle 0° of the transmitted light without diffusion or diffraction. The light is collected at an angle φ and the amount of light measured at this angle depends on the composition of the reaction system in the mini-reactor (droplet). Thus, microorganism growth changes the signal.

The method of the present invention allows a significant efficacy increase (due to the parallelization of a multitude of microbiological tests evaluating the influence of chemical substances on microorganisms. Due to the small sample volume needed (each droplet is one reactor) the amount of sample needed is very small which is another advantage of the invention.

In the case of the using tubes as outlined above, it is possible to fix a multiplicity of tubes (each tube containing a multiplicity of mini-reactors or mini-incubators in the form of droplets) on a support which can be rotated and thus allows presenting each mini-reactor to the detection device at defined intervals of time. This allows very precise measurements with the possibility of differentiating and discriminating small concentration effects of the substance ; the increase of the replicates will allows us to increase the robustness of statistical evaluations

The method of the present invention is suitable for determining and monitoring the influence of a broad variety of chemical substances on microorganisms. Encapsulation of microbial populations into droplets and monitoring their behaviour is of interest for fundamental microbiology as well as for determining the efficacy of antibiotics or antimicrobial agents on bacterial isolates. This may involve the determination of the minimum inhibitory concentration of an antibiotic which is possible with the method of the present invention in a more reliable way than with the usual screening test using microtitre plates where quantitative measurements are very difficult.

The method of the present invention allows automated determination with an unprecedented accuracy and with high performance.

Furthermore the method due to the very low sample volumes and corresponding to low material costs is also economically advantageous.

Due to the immobilization of the mini-reactors, growth behaviour can be reliably and reproducably monitored over many generations or cell cycles.

Furthermore, the possibility to carry out quantitative population measurements of microorganisms is an additional advantage of the method in accordance with the present invention.

## Claims

1. A method for determining the effect of chemical substances on microorganisms in miniaturized devices comprising the steps of
a) forming aqueous droplets comprising the microorganisms, a nutrient system and the chemical substance in various concentrations outside the miniaturized device,
b) forming a one-dimensional train of said droplets in a hydrophobic oil carrier medium wherein the droplets are isolated from each other,
c) transferring the one dimensional train of droplets isolated from each other into a miniaturized device thereby immobilizing the droplets in the device,
d) repeatedly measuring a signal indicative and representative of the microorganism growth for each of the isolated droplets in defined time intervals.

2. Method in accordance with claim 1 wherein the droplets are formed by mixing three different product streams with suitable injection means in a cross junction.

3. Method in accordance with claim 1 or 2 wherein the one dimensional train of droplets is formed by mixing the droplets formed with the hydrophobic oil in a suitable mixing chamber allowing the formation of discrete aqueous droplets isolated from each other in the hydrophobic oil carrier.

4. Method in accordance with any of claims 1 to 3 wherein the signal indicative of microorganism growth is a fluorescence signal.

5. Method in accordance with any of claims 1 to 3 wherein the signal indicative of microorganism growth is a light diffusion signal.

6. Method in accordance with any of claims 1 to 4 wherein the signal indicative of microorganism growth is a florescence signal combined with an absorbance signal.

7. Method in accordance with any of claims 1 to 4 wherein the signal indicative of microorganism growth is an optical signal, in particular a bright field imaging signal.

8. Method in accordance with any of claims 1 to 7 wherein the hydrophobic oil carrier is a fluorinated oil.

9. Method in accordance with claim 8 wherein the fluorinated oil is a perfluoropolyether (PFPE) oil or lubricant.

10. Method in accordance with any of claims 1 to 9 wherein the miniaturized device is a sealed tube with a diameter in the range of from 0.05 to 5 mm.

11. Method in accordance with any of claims 1 to 9 wherein the miniaturized device is a microfluidic chip with micro-channels with cross-sections in the range of from 1x1 µm² to 500x500 µm².

12. Use of the method in accordance with any of claims 1 to 11 for quantitative population measurements of microorganisms.
